# EUROPEAN PATENT APPLICATION

(11) **EP 0 835 654 A1**
(43) Date of publication of application: **15.04.1998**
(21) Application number: 97114849.9
(22) Date of filing: 27.08.1997
(51) Int. Cl.: A61K 9/20, A61K 9/48

(54) **Glucomannan-containing orally administered pharmaceutical preparations with sustained effect**

(30) Priority: 11.10.1996 JP 289380/96
(71) Applicant: Shimizu Chemical Corporation, Mihara-shi, Hiroshima-ken (JP)
(72) Inventor: Shibata, Kazuhide, Suita-shi, Osaka-fu (JP)
(74) Representative: Kinzebach, Werner, Dr.

(57) **Abstract**

An orally administered pharmaceutical preparation for a pharmacologically active ingredient contains an effective amount of said active ingredient and 0.5 to 3 g of glucomannan in a dosage form for a single oral administration. The preparation provides sustained effect of the active ingredient by delaying absorption of the active ingredient in the small intestine and enabling full absorption of residual part of the active ingredient in the large intestine.

## Description

### Field of the Invention

The present invention relates to a pharmaceutical preparation which allows to control the absorption rate of a pharmacologically active ingredient, and more particularly, to a pharmaceutical preparation which, by making use of glucomannan, allows to control the absorption rate of a pharmacologically active ingredient.

### Background of the Invention

With regard to pharmaceutical preparations for oral administration such as tablets, capsules and the like, there are a variety of pharmaceutical designs to reduce risks of side effects due to a rapid increase of blood levels of a drug and to allow full exhibition of the effect of a drug, by maintaining proper blood levels of the drug for a prolonged time. To achieve this purpose, what is generally employed is to control the release rate of the drug from a preparation to thereby indirectly control its absorption rate in the gastrointestinal tract. The control of the release rate of drugs are generally realized by delaying the release of the drug from the preparation.

If a pharmaceutical preparation passes through the gastrointestinal tract and is excreted out of the body prior to complete release from the preparation, part of the administered drug would not be utilized, and the bioavailability of the drug would thus be lowered. Therefore, it is important for said type of pharmaceutical preparation to ensure that the release of a drug from the preparation is completed within the gastrointestinal tract.

The inventor tried to develop a different type of pharmaceutical preparation which, instead of suppressing the release of a drug from a preparation, suppresses the conveyance of gastrointestinal content containing the drug once released from the preparation through the gastrointestinal tract, and also suppresses the diffusion of the drug once released from the preparation in the gastrointestinal content, and, in addition, removes the suppression in the large intestine to thereby accomplish absorption of the drug before excretion from the gastrointestinal tract.

### Summary of the Invention

Glucomannan is the chief polysaccharide present in the corms of a variety of Amorphophallus plants. The inventor noted that glucomannan makes a very viscous solution when dissolved in water and that glucomannan is not decomposed in the small intestine but is decomposed in the large intestine by Escherichia coli and loses its viscosity. The inventor then sought the possibility of properly delaying absorption of a drug in the small intestine both by suppressing conveyance of the drug through the small intestine in the direction to the anus and suppressing diffusion of the drug in the solution, both making use of the viscosity of the solution. As a result, the inventor has found that when glucomannan is orally administered together with a drug, gradual absorption of the drug occurred over a prolonged time period compared with the case without administration of glucomannan, and that there is no reduction of overall absorption of the drug.

Thus, the present invention provides a pharmaceutical preparation with sustained effect for oral administration of a pharmacologically active ingredient comprising a mixture of said pharmacologically active ingredient and glucomannan. In accordance with the present invention, the length of time during which the absorption of the pharmacologically active ingredient takes place may be conveniently controlled by simply adjusting the proportion of glucomannan to the amount of the active ingredient. Thus, the present invention allows sustainment of pharmacological effect of a variety of active ingredients orally administered to mammals including human.

Therefore, the present invention also provides a method of producing a pharmaceutical preparation with sustained effect for oral administration of a pharmacologically active ingredient, which method comprises admixing a predetermined amount of glucomannan with a predetermined amount of said pharmacologically active ingredient.

### BRIEF DESCRIPTION OF THE DRAWINGS

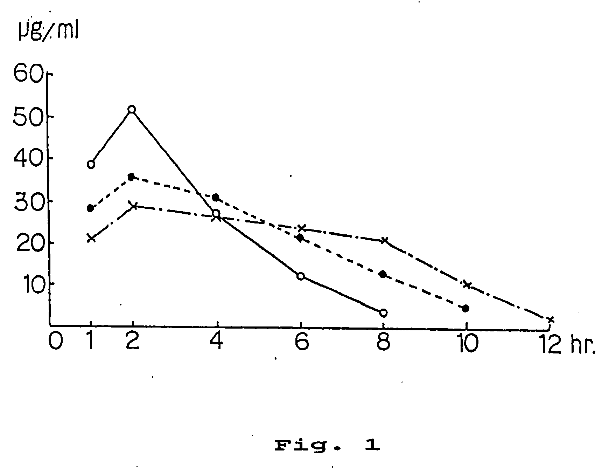

Figure 1 is a graph showing the profile of the blood concentration of nalidixic acid over time. In the figure, the horizontal axis represents the time length (in hours) after oral administration of the present pharmaceutical preparation containing nalidixic acid as a pharmacologically active ingredient. The vertical axis represent the concentration of nalidixic acid in blood. The line ○ ―○ represents the Control Test, ●―● Investigated Drug Test 1, and X―X Investigated Drug Test 2.

### Detailed Description of the Invention

In an embodiment of the present invention, a pharmaceutical preparation with sustained effect for oral administration of a pharmacologically active ingredient is provided which contains an effective amount of said pharmacologically active ingredient and 0.5 to 3 g of glucomannan in a dosage form for a single oral administration.

In an embodiment of the present invention, the method of producing a pharmaceutical preparation with sustained effect for oral administration of a pharmacologically active ingredient comprises admixing 0.5 to 3 g of glucomannan per an effective amount of said pharmacologically active ingredient.

The term an "effective amount" as used herein refers to the amount administered with the intention of producing an effect in treatment of prophylaxis of a disease, and more specifically, to the amount of the pharmacologically active ingredient contained in a recommended amount for a single administration of the pharmaceutical preparation containing the pharmacologically effective ingredient. Such an amount for a single administration may be defined (in some cases, in accordance with strata of the age of patients) on, for example, product inserts, in terms of the number of tablets or capsules, the weight or powders or granules, or the volume of liquid preparations.

The term a "dosage form for a single oral administration" as used herein is a notion including the amount of pharmaceutical preparation administered, such as the number of tablets or capsules, the weight of powders or granules, or the volume of liquid preparations, which correspond to the "effective amount" of the active ingredient.

As for pharmacologically active ingredients, any ingredients that are desired to be absorbed in the gastrointestinal tract may be used. Examples of such ingredients include, but not limited to, chemotherapeutic agents such as antibacterials, antibiotics, antipyretic analgesics, and psychotropic drugs. However, any other drug may be used for which it is desired to undergo sustained absorption in the gastrointestinal tract. Also, it is possible to combine two or more pharmacologically active ingredients with similar or different activities.

The present pharmaceutical preparation may be in any convenient form suitable to oral administration, such as a powder, granules, capsules, tablets, an aqueous preparation (which includes a gel-type preparation) or the like. It may be possible to add excipients such as corn starch, coloring agents, aromatic agents and flavoring agents in the case of a powder, and also binders in the case of granules. In the case of capsules, excipients, coloring agents and the like may be added if desired, in addition to glucomannan and a pharmacologically active ingredient. In the case of tablets, excipients, binders, disintegrants, lubricants and the like may be added. In the case of aqueous preparations, flavoring agents, aromatic agents and the like may be added. These preparations may be produced in accordance to conventional methods.

After oral administration, the pharmaceutical preparations of the present invention make a highly viscous solution, the viscosity of which is maintained while the solution passes through the small intestine. Thus, the conveyance of the solution through the small intestine in the direction to the anus is delayed, and, at the same time, the diffusion of the pharmacologically active ingredient in the solution is suppressed. Because of these two effects, absorption of the active ingredient is delayed, resulting in sustained absorption. However, as glucomannan undergoes decomposition by E. coli in the large intestine and its solution thus loses viscosity, residual part of the pharmacologically active ingredient in the glucomannan solution will then diffuse freely in the large intestine and eventually be absorbed. Therefore, the suppression of absorption of pharmacologically active ingredient according to the present invention only delays the absorption and does not reduce the overall amount to be absorbed. Thus, the pharmacologically active ingredient administered will be gradually but fully absorbed over a prolonged period of time. With the higher proportion of glucomannan used, the more delayed is the absorption, resulting in greater sustainment. On the contrary, with the lower proportion of glucomannan, delaying effect on absorption is reduced. Therefore, the length of time during which the absorption of the pharmacologically active ingredient takes place may be conveniently controlled by adjusting the proportion of glucomannan.

### Pharmacological Test

The following clinical test was carried out using nalidixic acid as a pharmacologically active ingredient.

### Subjects and Method:

Five male normal subjects (mean age: 30.4 years old) were used in the test. The whole test was divided into three sub-tests, i.e. Control Test, Investigated Drug Test 1 and Investigated Drug Test 2, and these tests were performed on the same subjects. Of these three sub-tests, Control Test was carried out first, and after a one-week washout period, Investigated Drug Test 1 was performed, and then, again after a on-week washout period, Investigated Drug Test 2 followed.

For Control Test, the subjects orally received four control capsules as shown below (therefore, 1.0 g of nalidixic acid and 1.0 g of starch) after breakfast with 200 ml of water.

### (Control capsule)

The following ingredients were admixed and filled in a capsule container to give a capsular preparation.

| | |
|---|---|
| Nalidixic acid | 250 mg |
| Starch | 250 mg |
| Total | 500 mg |

For Investigated Drug Test 1, the subjects orally received four capsules of Example 1 (therefore, 1.0 g of nalidixic acid and 1.0 g of glucomannan) with 200 ml of water. For Investigated Drug Test 2, the subjects orally received eight capsules of Example 2 (therefore 1.0 g of nalidixic acid, 2.0 g of glucomannan and 1.0 g of starch) with 200 ml of water. After breakfast, the subjects were allowed to spend the day as usual. In every test, blood sample were taken 1, 2, 4, 6, 8, 10 and 12 hours after the breakfast, immediately followed by determination of the drug level in the blood samples.

### Test Results:

Fig. 1 shows the profile of blood concentration of nalidixic acid after administration of the pharmaceutical preparations. Table 1 shows the length of time during which blood concentration of nalidixic acid is maintained to be equal to or higher than 20 µg/ml and 10 µg/ml, respectively, as well as the highest blood concentration of nalidixic acid for each preparation. As Table 1 shows, while the length of time during which the predetermined blood concentrations of the pharmacologically active ingredient is maintained is greatly prolonged by the use of glucomannan, the blood concentration curve produced a smooth form with expanded skirts as a result of lowering of the highest blood concentration. In addition, as shown in Table 2, there was no significant difference between the areas under the curves in Fig. 1 for the respective test. This indicates that glucomannan does not hinder the overall final absorption of the pharmacologically active ingredient.

**TABLE 1**

| Lengths of time during which blood concentration of nalidixic acid is maintained, and the highest blood concentrations. | | | |
|---|---|---|---|
| Blood Concentration | Length of Time | | |
| | GM 0 g | GM 1.0 g | GM 2.0 g |
| ≧ 20 µg/ml | 4 | 6 | 8 |
| ≧ 10 µg/ml | 6 | 8 | 10 |
| Highest Blood Concentration (µg/ml) | 51.26 | 34.94 | 29.46 |

In the table, GM represents glucomannan.

**TABLE 2**

| Influence of glucomannan on the area under the curve (µg/ml) | |
|---|---|
| Test | Area under the Curve |
| Control Test (GM 0 g) | 135.54 |
| Investigated Drug Test 1 (GM 1.0 g) | 132.70 |
| Investigated Drug Test 2 (GM 2.0 g) | 138.96 |

It is clear that the above described effect of glucomannan on gastrointestinal absorption of the drug will be reproducible not only with nalidixic acid but also with any other drugs, for that effect is based upon physical properties, i.e. delayed conveyance through the small intestine due to the viscosity of glucomannan solution as well al the suppression of drug diffusion due to the viscosity.

### Examples

### (Capsules 1)

The following ingredients were admixed and filled in a capsule container to give a capsular preparation.

| | |
|---|---|
| Nalidixic acid | 250 mg |
| Glucomannan | 250 mg |
| Total amount | 500 mg |

### (Capsules 2)

The following ingredients were admixed and filled in a capsule container to give a capsular preparation.

| | |
|---|---|
| Nalidixic acid | 125 mg |
| Glucomannan | 250 mg |
| Starch | 125 mg |
| Total amount | 500 mg |

## Claims

1. A pharmaceutical preparation with sustained effect for oral administration of a pharmacologically active ingredient, wherein said preparation comprises a mixture of said pharmacologically active ingredient and glucomannan.

2. The pharmaceutical preparation of claim 1, wherein said preparation contains an effective amount of said pharmacologically active ingredient and 0.5 to 3 g of glucomannan in a dosage form for a single oral administration.

3. The pharmaceutical preparation of claim 1 or 2, wherein said pharmacologically active ingredient is a chemotherapeutic drug, an antibiotic, an antipyretic analgesic, an antiinflammatory and/or a psychotropic drug.

4. A method of producing a pharmaceutical preparation with sustained effect for oral administration of a pharmacologically active ingredient, which method comprises admixing a predetermined amount of said pharmacologically active ingredient with a predetermined amount of glucomannan.

5. The method of claim 4 comprising admixing 0.5 to 3 g of glucomannan per an effective amount of said pharmacologically active ingredient.

6. The method of claim 4 or 5, wherein said pharmacologically active ingredient is a chemotherapeutic drug, an antibiotic, an antipyretic analgesic, an antiinflammatory and/or a psychotropic drug.
